# EUROPEAN PATENT SPECIFICATION

(11) **EP 3 901 962 B1**
(45) Date of publication and mention of the grant of the patent: **15.07.2026**
(21) Application number: 20170858.3
(22) Date of filing: 22.04.2020
(51) Int. Cl.: G16H 30/40, G16H 40/20, G16H 50/70

(54) **TOOL AND METHOD TO ANALYSE MEDICAL IMAGES OF PATIENTS**
WERKZEUG UND VERFAHREN ZUR ANALYSE VON MEDIZINISCHEN BILDERN VON PATIENTEN
OUTIL ET PROCÉDÉ POUR ANALYSER DES IMAGES MÉDICALES DE PATIENTS

(43) Date of publication of application: 27.10.2021
(73) Proprietor: AITEM - Artificial Intelligence Technologies Multipurpose S.r.l., 10129 Torino (IT)
(72) Inventor: TRICARICO, Davide, 10146 Torino (IT); LONGO, Simone, 10022 Carmagnola (IT); GIROTTO, Marco, 10131 Torino (IT); MELIS, Massimiliano, 10098 Rivoli (IT)
(74) Representative: Bruni, Giovanni

(56) References cited:
- WO-A1-2015/134530
- US-A1- 2019 267 133
- US-A1- 2019 371 439

## Description

The current invention relates to a tool to analyse medical images of patients, using a database comprising preloaded medical images.

Specifically, the tool is designed to analyse medical images of patients by using an algorithm.

In healthcare, time, especially wasted time, contributes to the overcrowding of hospitals, anxious patients and a reduced outcome of the patients.

It is commonly accepted that the sooner a patient gets the right care in the right place with the right resources, the higher the chances are to obtain a positive outcome.

The value of time is highly associated with patients' lives in healthcare.

A known problem in healthcare are the inefficiencies in the process starting from the first appearance of symptoms of a patient to be examined by the right doctor with the right expertise until obtaining optimal care. Very often, valuable time is already wasted from even before a patient has been diagnosed with a certain disease due to, for example, the complexity of the disease, long waiting lists to visit certain expert doctors, et cetera.

Furthermore, it is generally known that these inefficiencies increase the cost of proper healthcare dramatically.

Document WO 2015/134530 Al discloses methods and a personalized system for retrieving similar image-based subject data. A method for adaptive learning of imaging data comprises accessing a subject database comprising subject imaging data. Next a search of the subject database is conducted using one or more search parameters which can include one or more image data associated with a subject undergoing treatment. Next at least one match of the search can be provided on a user interface.

Document US 2019/267133 Al discloses an appointment scheduling device for scheduling an appointment for a patient to visit a health provider includes an embedder a predictor and a scheduler. The embedder receives input data about the patient. The input data is associated with a request to schedule the appointment with the health provider. The embedder generates an embedding based on the input data. The predictor receives the embedding and predicts an appointment parameter based on the embedding. The scheduler schedules the appointment based on the appointment parameter.

Finally, document US 2019/371439 Al discloses an apparatus for determining similarity between medical data sets for a plurality of patients or other subjects comprises at least one data store and a processing resource.

The purpose of the current invention is to provide a solution to the aforementioned and other disadvantages.

The current invention relates to a tool to analyse medical images of patients as defined in appended claim 1**,** the tool using a database comprising preloaded medical images, whereby the tool has an input interface for loading a plurality of medical images of different patients to be analysed, wherein the tool is provided with an algorithm to prioritise patients of which the medical images have been loaded according to the similarity between their medical images to be analysed and the preloaded medical images already provided in the database, for generating a disease similarity index, wherein the said index is used to rank and prioritise the handling of patients, the patients of which the loaded medical images have the highest similarity index are ranked with the highest priority.

An advantage of a tool according to the invention is that it provides an easy and fast manner to guide healthcare practitioners to decide which patients should be handled first due to a higher priority proposed by the tool, for example, to perform extra tests to diagnose patients with a certain disease or referring patients immediately to a doctor with the necessary expertise for optimal care.

The algorithm used to prioritise patients is based on the number of cases for the most relevant disease weighted by their computed similarity for each image to be analysed, multiplied by a tunable coefficient K and by the number of cases for other relevant diseases and divided by a tunable parameter Rmax.

In a preferred embodiment the tunable coefficient K is determined by an interactive iterative process wherein a subset of selected data preloaded in the database is chosen as a validation set and wherein the remaining preloaded data in the database are used as a training set, and wherein at the start a value for the tunable coefficient K is chosen and then running the algorithm to prioritise the
medical images in the validation set using data from the training set and investigating if the performance of the prioritisation is acceptable, and in case in which the performance is not acceptable to repeat this algorithm each time with a different value for the coefficient K until an acceptable performance is achieved.

In yet another preferred embodiment, the tunable parameter Rmax is determined, depending on the obtained value of the tunable coefficient K, in such a way that the similarity index is limited to a value of about one hundred percent.

The preloaded medical images provided in the database are preferably present in the database in the form of a semantical representation and the medical images of different patients to be analysed are first converted into a semantical representation in order to be compared with the semantical representations of the preloaded medical images in the database by means of cosine similarity which has been proven to give optimal results.

The semantical representations of medical images are achieved by a truncated deep neural network like DenseNet 121 and reduced by average pooling, which generates a 1024-components vector.

An advantage of this is proven practise.

Furthermore, the tool can be used to prioritise patients according to the plausible severity of a certain disease predicted by the tool.

An advantage of this is that patients potentially suffering from a certain disease with a higher severity can be examined and/or treated by the doctor first.

The database preferably contains medical images of patients taken at different stages of progression of a certain disease, and that the tool for each stage of progression provides the medical images of the best and worst possible cases, allowing the comparison of the progression of the disease of a patient with the best and worst case of the preloaded medical images in the database.

This allows to evaluate progression of the patient and of the treatment of the disease compared to the best and worst possible outcome, for example to evaluate the effectiveness of a treatment for the disease in concern.

The medical images used by the tool need to be in a certain format to obtain optimal results and therefore the medical images can be preprocessed by removal of annotations, resizing the image to l000xl000 pixels and normalization by the mean and the standard deviation.

In a preferred embodiment the tool is accommodated with an input interface to upload medical images from an external platform at distance, for example from a computer in a hospital or the like.

The invention also relates to a method to analyse medical images of patients, as defined in appended claim 9.

With the intention of better showing the characteristics of the invention, a tool and a method to analyse medical images is described hereinafter, by way of an example without any limiting nature, with reference to the accompanying drawings wherein:
figure 1 demonstrates a schematic representation of a tool according to the invention;
figure 2 shows a technical overview with an output of a similarity analysis according to the invention;
figure 3 shows a schematic representation of a similarity algorithm according to the invention;
figure 4 shows an overview of the priority algorithm according to the invention;
figure 5 shows a schematic representation to obtain a tunable coefficient K and a tunable parameter Rmax according to the invention;

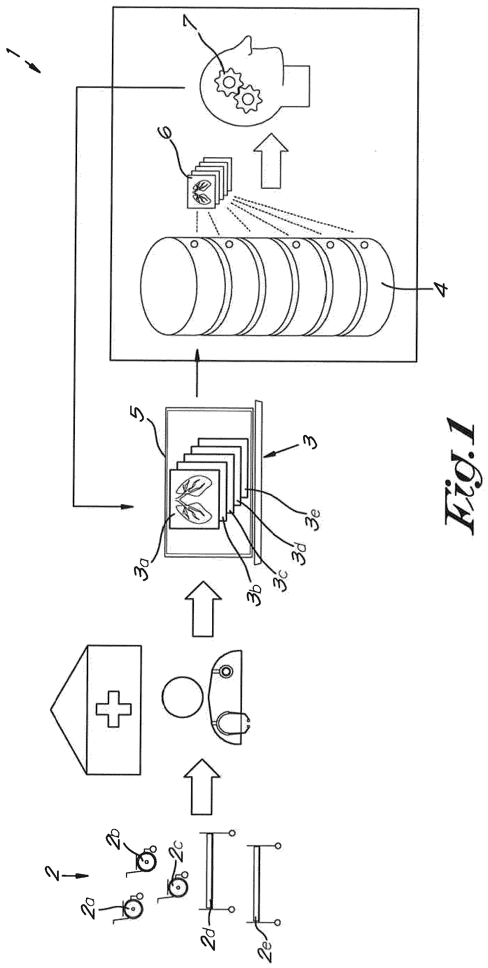

Figure 1 shows a schematic representation of an overview of the overall use of a tool 1 according to the invention.

For example, a patient 2A is advised to go to a hospital to obtain a medical image 3 of an area of interest, such as but not limiting to an X-ray or a computerised tomography scan, which is anonymised by eliminating personal information, randomised and stored in a database 4 at the hospital to ensure records can be tracked by healthcare practitioners.

The medical image 3A of this patient 2A can be analysed by the before mentioned tool 1 containing a suitable input interface to upload medical images 3 from an external platform 5 at the hospital, for example from a computer or a digital network in the hospital or the like.

The medical image 3 is analysed by using several algorithms 7, which analysis can afterwards be coupled to the medical image 3 when stored in the database 4 as well as the final diagnosis and treatment of the patient.

The medical image 3A of the patient 2A to be analysed can be compared by similarity to the preloaded medical images 6 present in the database 4.

Medical images 3A, 3B, 3C, 30, 3E of different patients 2A, 2B, 2C, 20, 2E to be analysed can then be prioritised by ranking the medical images 3 according to similarity with the preloaded medical images 6 and/or importance and/or severity.

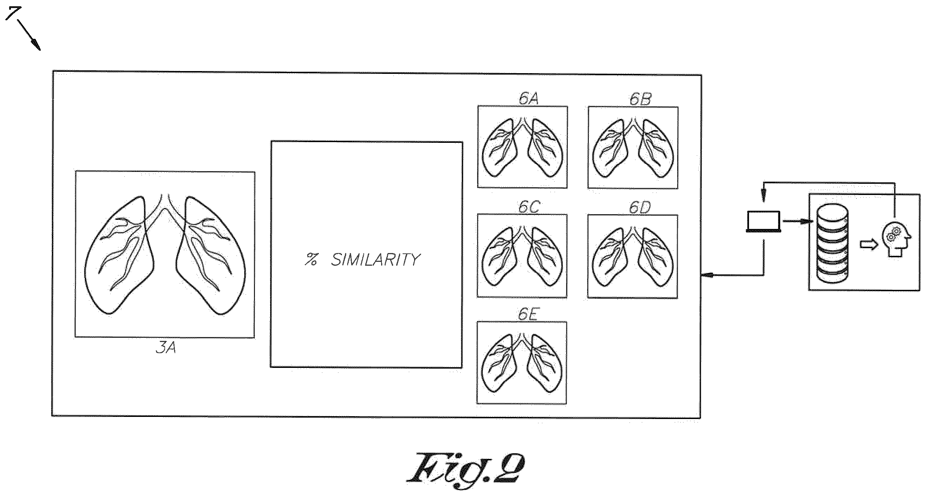

Figure 2 shows a technical overview and an output 7 of a similarity analysis. In order to perform a similarity analysis the medical images 3 are analysed by the similarity algorithm 8.

The preloaded medical images 6 provided in the database 4 are present in the database 4 in the form of a semantical representation 9 and the medical images 3 of different patients 2 to be analysed are first converted into their semantical representations 9.

After the convergence of the medical images 3 into their semantical representations 9, the semantical representations 9 of the medical images 3 of the patients 2 to be analysed are compared with the semantical representations 9 of the preloaded medical images 6 in the
database 4 by means of cosine similarity 10.

The output 7 of such a similarity analysis, as shown in figure 2, will provide a percentage of similarity to the five most similar cases 6A, 6B, 6C, 60, 6E found in the database 4 which will also be provided.

For example, the medical image 3A of patient 2A, in this case a computerized tomography scan of his lungs, undergoes such a similarity analysis. The output 7 provides a percentage of similarity, in this case eighty percent similarity, and the five most similar cases 6A, 6B, 6C, 6D, 6E present in the database 4. The tool 1 will also provide the final diagnosis of the patients from those most similar cases 6A, 6B, 6C, 60, 6E.

So, the similarity output 7 provides a healthcare practitioner with the following information: the medical image 3A of patient 2A is eighty percent similar to the five most similar cases, 6A, 6B, 6C, 60, 6E, and that the patients from these cases 6A, 6B, 6C, 60, 6E were
ultimately diagnosed with for example coronavirus disease 2019 and therefore the medical image 3A of patient 2A demonstrates eighty percent similarity to coronavirus disease 2019.

It should be noted that for each medical image 3 of patients 2 to be analysed, the five most similar cases 6 to which the medical image 3 will be compared by means of cosine similarity 10 can and probably will be different for each patient 2 of which a medical image 3 has been loaded in the tool 1.

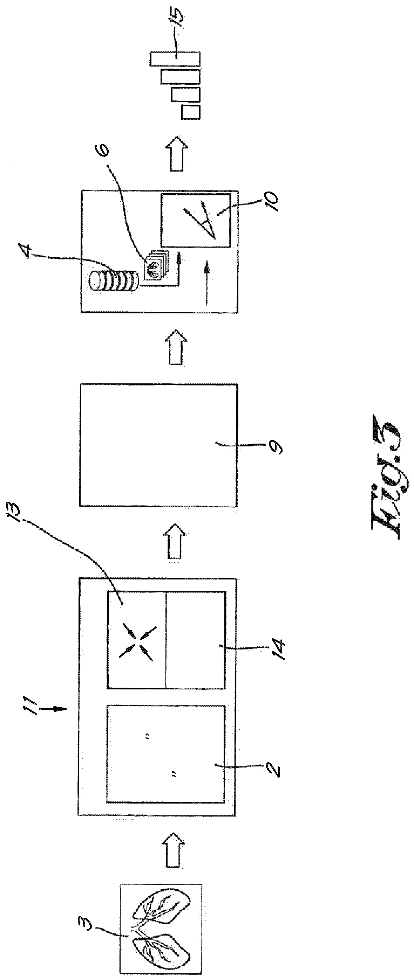

As shown in figure 3, the similarity algorithm 8 contains several steps. The medical images 3 of different patients 2 to be analysed can be preprocessed 11 by removal of annotations 12, resizing 13 the image to 1000x1000 pixels and normalisation 14 by the mean and the standard deviation.

Following the preprocessing 11, medical images 3 are converted to their semantical representations 9by a truncated deep neural network and reduced by average pooling in order to generate a multiple components vector, for example a 1024-components vector, which is then used for cosine similarity 10 analysis to provide a similarity ranking 15.

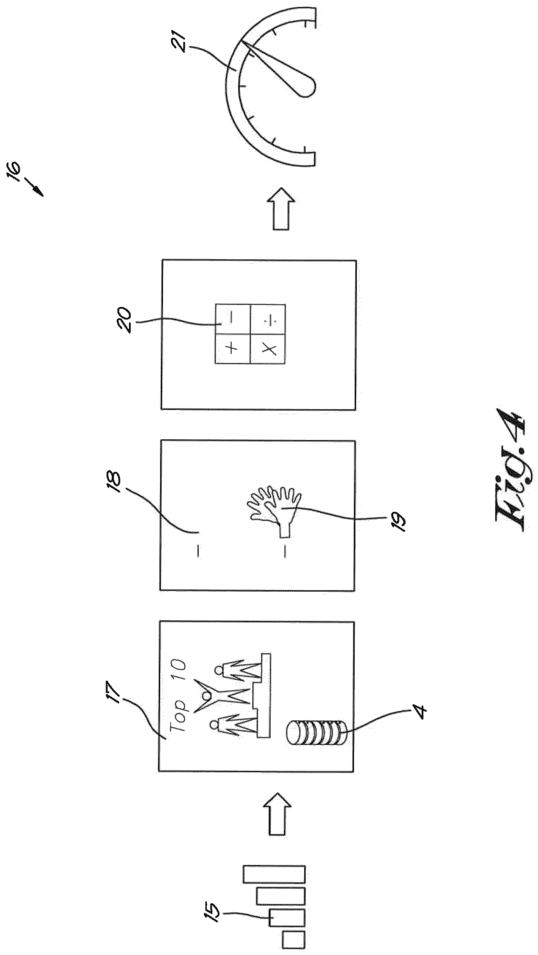

Figure 4 shows an overview to transform the similarity ranking 15 to a priority. In order to prioritise medical images 3 of different patients 2 to be analysed, the medical images 3 of those patients 2 are analysed by the priority algorithm 16.

Given the similarity analysis as described before, the preloaded medical images 6 present in the database 4 are ranked by similarity 15 after which the tool will select the top ten most similar cases 17 from the database 4.

In case of patient 2A of which a medical image 3A of a lung has been uploaded, the tool 1 will select the top ten most similar cases 17 related to lungs, not necessarily all related to the same disease but corresponding to the closest similar cases.

For each of these similar cases 17, the tool 1 will classify 18 the top ten most similar cases 17 according to their related diseases and count 19 the number of cases related to each disease within the top ten 17.

Following the above mentioned top ten most similar cases 17 for patient 2A, the tool can for example count 19 that five patients of these similar cases 17 were
reported to be diagnosed with coronavirus disease 2019, three with another form of pneumonia and two were healthy.

The tool will then perform a priority rating 20 based on the number of cases for the most relevant disease weighted by their computed similarity for each image 3 to be analysed multiplied by a tunable coefficient K, to which the number of cases for other relevant diseases is added and divided by a tunable parameter Rmax.

To illustrate this further, in case of patient 2A coronavirus disease 2019 can be considered the most relevant disease based on the top ten most similar cases 17 in this instance. In the priority rating 20 the number of coronavirus disease 2019 cases is multiplied by a tunable
coefficient K and added by the number of cases for other pneumonia and divided by a tunable parameter Rmax.

The outcome of priority algorithm 16 is a disease similarity index 21 for the most relevant disease, which in case of patient 2A could be a coronavirus disease 2019 similarity index.

The medical images 3 of different patients 2 to be analysed can then be ranked with the highest priority by means of disease similarity index 21.

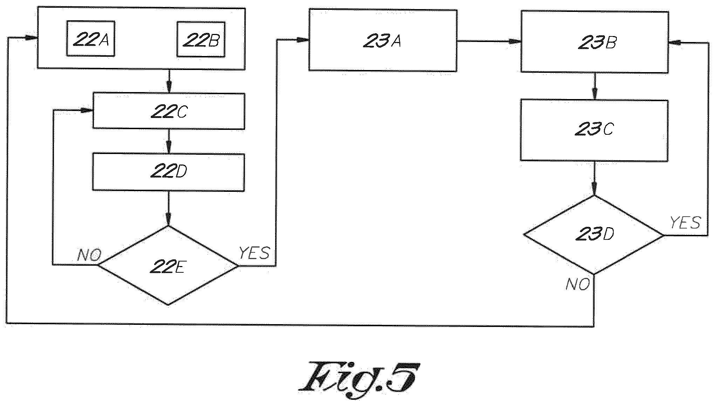

Figure 5 shows a method to obtain a value for the tunable coefficient K and a value for the tunable parameter Rmax.

The tunable coefficient K is determined by an interactive iterative process wherein a subset of selected data preloaded 6 in the database 4 is chosen as a validation set 22A and wherein the remaining preloaded data 6 in the database 4 are used as a training set 22B, and wherein at the start a value 22C for the tunable coefficient K is
chosen and then running the algorithm 16 to prioritise 220 the medical images 3 in the validation set 22A using data from the training set 22B and evaluating if the performance 22E of the prioritisation is acceptable, and in case the performance 22E is not acceptable to repeat the prioritisation algorithm 220 each time with a different value 22C for the coefficient K until an acceptable performance 22E is achieved.

When the performance 22E of the value 22C for the tunable coefficient K is acceptable, tunable parameter Rmax 23A is determined in such a way that the similarity index is limited to a value of about one hundred percent.

The tool 1 can also be used to prioritise patients 2 according to the plausible severity of a certain disease, in which case the patient would be examined by the doctor prior to less severe cases.

Furthermore, the tool 1 can be used to predict at which stage of progression of a certain disease a patient 2 is and can provide the worst and/or best similar medical images for the relevant disease, allowing healthcare practitioners to evaluate the effectiveness of the treatment of the particular patient and to how the disease could progress compared to best and worst case.

The current invention is by no means limited to the embodiments described as an example and shown in the drawings, but a tool to analyse medical images according to the invention can be realized in all kind of variants without departing from the scope of the invention as defined by the claims.

## Claims

1. Tool (1) to analyse medical images (3) of patients (2), comprising a database (4) having preloaded medical images (6) of patients, each preloaded image having been diagnosed with a disease, and an input interface for loading a plurality of medical images (3) of different patients (2) to be analysed,
wherein the tool (1) is provided with an algorithm (16) to prioritise patients to be analysed (2) of which the medical images (3) have been loaded according to the similarity between their medical images (3) to be analysed and the preloaded medical images (6) already provided in the database (4) for generating a disease similarity index (21),
wherein the said disease similarity index (21) is used to rank and prioritise the handling of patients (2),
the tool (1) being **characterised in that** the outcome of the algorithm (16) is a disease similarity index (21) for the most relevant disease,
wherein, to obtain the most relevant disease for a medical image (3) to be analysed, the tool (1) ranks the preloaded medical images (6) and selects the top ten most similar cases (17) from the database (4) and, for each of these similar cases (17), the tool (1) classifies (18) the top ten most similar cases (17) according to their related diseases and counts (19) the number of cases related to each disease within the top ten, the most relevant disease being the disease with the highest count;
and the tool (1) performs priority rating based on the number of cases for the most relevant disease weighted by their computed similarity for each image (3) to be analysed multiplied by a tunable coefficient K and by the number of cases for other relevant diseases and divided by a tunable parameter Rmax.

2. Tool (1) according to claim 1, wherein the tunable coefficient K is determined by an interactive iterative process wherein a subset of selected data preloaded 6 in the database 4 is chosen as a validation set 22A and wherein the remaining preloaded data 6 in the database 4 are used as a training set 22B, and wherein at the start a value 22C for the tunable coefficient K is chosen and then running the algorithm 16 to prioritise 220 the medical images 3 in the validation set 22A using data from the training set 22B and evaluating if the performance 22E of the prioritisation is acceptable, and in case in which the performance 22E is not acceptable to repeat the prioritisation algorithm 220 each time with a different value 22C for the coefficient K until an acceptable performance 22E is achieved.

3. Tool according to claim 2, wherein the tunable parameter Rmax 23A is determined, depending on the determined value 22C of the tunable coefficient K, in such a way that the similarity index (21) is limited to a value of one hundred percent.

4. Tool (1) according to claim 1, wherein the preloaded medical images (6) provided in the database (4) are present in the database (4) in the form of a semantical representation (9) and the medical images (3) of different patients (2) to be analysed are first transformed in to a semantical representation (9) in order to be compared with the semantical representations (9) of the preloaded medical images (6) in the database (4) by means of cosine similarity (10).

5. Tool (1) according to claim 4, wherein the semantical representations (9) of medical images (3) are achieved by a truncated deep neural network DenseNet 121 and reduced by average pooling, which generates a 1024-components vector.

6. Tool (1) according to any of the previous claims, wherein the tool (1) can be used to prioritise patients according to the plausible severity of a certain disease.

7. Tool (1) according to claim 1, wherein medical images (3) used by the tool (1) are preprocessed (11) by removal of annotations (12), resizing (13) the image to 1000x1000 pixels and normalization (14) by the mean and the standard deviation.

8. Tool (1) according to claim 1, wherein the input interface is suitable for loading medical images (3) from an external platform at distance.

9. Method to analyse medical images (3) of patients (2) suitable for use in a tool (1) according to any of the previous claims, the method making use of a database (4) with preloaded medical images (6) and an algorithm (16) to prioritise patients to be analysed (2); wherein the method is used to prioritise said medical images (3) of different patients (2) to be analysed and the method comprising the following steps:
- loading the medical images (3) of the patients (2) to be prioritised in the tool (1);
- comparing each of the loaded images (3) of different patients (2) with the preloaded medical images (6) to determine a disease similarity index (21); and,
- ranking by highest priority each loaded image (3) of the patients (2) according to their disease similarity index (21),
the method being **characterised in that** the outcome of algorithm (16) is a disease similarity index (21) for the most relevant disease
wherein, to obtain the most relevant disease for a medical image (3) to be analysed, the tool (1) ranks the preloaded medical images (6) and selects the top ten most similar cases (17) from the database (4) and, for each of these similar cases (17), the tool (1) classifies (18) the top ten most similar cases (17) according to their related diseases and counts (19) the number of cases related to each disease within the top ten, the most relevant disease being the disease with the highest count;
and the tool (1) is based on the number of cases for the most relevant disease weighted by their computed similarity for each image (3) to be analysed multiplied by a tunable coefficient K and by the number of cases for other relevant diseases and divided by a tunable parameter Rmax.

10. Method according to claim 9, wherein the step of comparing medical images (3) of different patients (2) with preloaded medical images (6) of a database (4) based on similarity comprises the following steps:
- preloaded medical images (6) in the database (4) are transformed to a semantical representation (9) of each medical image (6);
- medical images (3) of different patients (2) to be analysed are loaded into the tool (1);
- medical images (3) of different patients (2) to be analysed are converted into a semantical representation (9); and,
- the semantical representations (9) of the preloaded medical images (6) in the database (4) and the semantical representations (9) of the medical images (3) of different patients (2) to be analysed are compared by means of cosine similarity (10).

11. Method according to claim 10, wherein the step of converting medical images (3, 6) into semantical representations (9) comprises the following steps:
- medical images (3, 6) are loaded into a database (4) or the tool (1);
- medical images (3, 6) are elaborated by a truncated deep neural network DenseNet 121; and,
- medical images (3, 6) are reduced by average pooling, which generates a 1024-components vector.

## Patentansprüche

1. zur Analyse medizinischer Bilder (3) von Patienten (2), bestehend aus einer Datenbank (4) mit vorab geladenen medizinischen Bildern (6) von Patienten, wobei jedem vorab geladenen Bild eine Krankheit diagnostiziert wurde, und einer Eingabeschnittstelle zum Laden mehrerer medizinischer Bilder (3) verschiedener Patienten (2) zur Analyse,
wobei das Werkzeug (1) verfügt über einen Algorithmus (16) zur Priorisierung der zu analysierenden Patienten (2), deren medizinische Bilder (3) geladen wurden, anhand der Ähnlichkeit zwischen ihren zu analysierenden medizinischen Bildern (3) und den bereits in der Datenbank (4) vorhandenen vorab geladenen medizinischen Bildern (6). Der Algorithmus generiert einen Krankheitsähnlichkeitsindex (21),
wobei dieser Krankheitsähnlichkeitsindex (21) dient zur Rangfolge und Priorisierung der Behandlung der Patienten (2),
das Werkzeug (1) **dadurch gekennzeichnet ist, dass** das Ergebnis des Algorithmus (16) ein Krankheitsähnlichkeitsindex (21) für die relevanteste Krankheit ist,
wobei um die relevanteste Krankheit für ein zu analysierendes medizinisches Bild (3) zu ermitteln, Werkzeug (1) sortiert die vorab geladenen medizinischen Bilder (6) und wählt die zehn ähnlichsten Fälle (17) aus der Datenbank (4) aus, und für jeden dieser Fälle (17) klassifiziert Werkzeug (1) die zehn ähnlichsten Fälle (17) nach ihren zugehörigen Krankheiten und zählt (19) die Anzahl der Fälle jeder Krankheit unter den zehn ähnlichsten Fällen, die Krankheit mit der höchsten Fallzahl gilt als die relevanteste, und
das Werkzeug (1) führt eine Prioritätsbewertung durch, basierend auf der Anzahl der Fälle der relevantesten Krankheit, gewichtet mit ihrer berechneten Ähnlichkeit für jedes zu analysierende Bild (3), multipliziert mit einem einstellbaren Koeffizienten K und der Anzahl der Fälle anderer relevanter Krankheiten und dividiert durch einen einstellbaren Parameter Rmax.

2. Werkzeug (1) nach Anspruch 1, wobei der einstellbare Koeffizient K durch einen interaktiven iterativen Prozess bestimmt wird, bei dem eine Teilmenge der in der Datenbank 4 vorab geladenen Daten 6 als Validierungsset 22A und die restlichen vorab geladenen Daten 6 in der Datenbank 4 als Trainingsset 22B verwendet werden, und wobei zu Beginn ein Wert 22C für den einstellbaren Koeffizienten K gewählt wird und anschließend der Algorithmus 16 ausgeführt wird, um die medizinischen Bilder 3 im Validierungsset 22A mithilfe von Daten aus dem Trainingsset 22B zu priorisieren und zu bewerten, ob die Leistung 22E der Priorisierung akzeptabel ist, und falls die Leistung 22E nicht akzeptabel ist, der Priorisierungsalgorithmus 220 jedes Mal mit einem anderen Wert 22C für den Koeffizienten K wiederholt wird, bis eine akzeptable Leistung 22E erreicht ist.

3. Werkzeug nach Anspruch 2, wobei der einstellbare Parameter Rmax 23A in Abhängigkeit vom ermittelten Wert 22C des einstellbaren Koeffizienten K so bestimmt wird, dass der Ähnlichkeitsindex (21) auf einen Wert von einhundert Prozent begrenzt ist.

4. Werkzeug (1) nach Anspruch 1, wobei die in der Datenbank (4) bereitgestellten vorab geladenen medizinischen Bilder (6) in der Datenbank (4) in Form einer semantischen Repräsentation (9) vorliegen und die zu analysierenden medizinischen Bilder (3) verschiedener Patienten (2) zunächst in eine semantische Repräsentation (9) transformiert werden, um sie mittels Kosinusähnlichkeit (10) mit den semantischen Repräsentationen (9) der vorab geladenen medizinischen Bilder (6) in der Datenbank (4) zu vergleichen.

5. Werkzeug (1) nach Anspruch 4, wobei die semantischen Repräsentationen (9) von medizinischen Bildern (3) durch ein verkürztes tiefes neuronales Netzwerk DenseNet 121 erreicht und durch Average Pooling reduziert werden, wodurch ein Vektor mit 1024 Komponenten erzeugt wird.

6. Werkzeug (1) nach einem der vorhergehenden Ansprüche, wobei das Werkzeug (1) zur Priorisierung von Patienten nach dem wahrscheinlichen Schweregrad einer bestimmten Erkrankung verwendet werden kann.

7. Werkzeug (1) nach Anspruch 1, wobei die vom Werkzeug (1) verwendeten medizinischen Bilder (3) durch Entfernen von Annotationen (12), Skalieren (13) des Bildes auf 1000x1000 Pixel und Normalisieren (14) mittels Mittelwert und Standardabweichung vorverarbeitet (11).

8. Werkzeug (1) nach Anspruch 1, wobei die Eingabeschnittstelle zum Laden medizinischer Bilder (3) von einer externen Plattform geeignet ist.

9. Verfahren zur Analyse medizinischer Bilder (3) von Patienten (2), die zur Verwendung in einem Werkzeug (1) nach einem der vorhergehenden Ansprüche geeignet sind, wobei das Verfahren eine Datenbank (4) mit vorab geladenen medizinischen Bildern (6) und einen Algorithmus (16) zur Priorisierung der zu analysierenden Patienten (2) verwendet. wobei das Verfahren dazu dient, die medizinischen Bilder (3) verschiedener Patienten (2) für die Analyse zu priorisieren, und das Verfahren die folgenden Schritte umfasst:
- Laden der medizinischen Bilder (3) der zu priorisierenden Patienten (2) in das Tool (1);
- Vergleich jedes der geladenen Bilder (3) verschiedener Patienten (2) mit den vorab geladenen medizinischen Bildern (6) zur Bestimmung eines Ähnlichkeitsindex (21) für die Erkrankung; und,
- Rangfolge der geladenen Bilder (3) der Patienten (2) nach höchster Priorität gemäß ihrem Krankheitsähnlichkeitsindex (21),
das Verfahren **dadurch gekennzeichnet ist, dass** das Ergebnis des Algorithmus (16) ein Krankheitsähnlichkeitsindex (21) für die relevanteste Krankheit ist
wobei um die relevanteste Krankheit für ein zu analysierendes medizinisches Bild (3) zu ermitteln, ordnet das Tool (1) die vorgeladenen medizinischen Bilder (6) und wählt die zehn ähnlichsten Fälle (17) aus der Datenbank (4) aus. Für jeden dieser ähnlichen Fälle (17) klassifiziert das Tool (1) die zehn ähnlichsten Fälle (17) gemäß ihren zugehörigen Krankheiten und zählt (19) die Anzahl der Fälle, die mit jeder Krankheit unter den zehn ähnlichsten Fällen zusammenhängen. Die relevanteste Krankheit ist diejenige mit der höchsten Anzahl,
und das Werkzeug (1) basiert auf der Anzahl der Fälle für die relevanteste Krankheit, gewichtet mit ihrer berechneten Ähnlichkeit für jedes zu analysierende Bild (3), multipliziert mit einem einstellbaren Koeffizienten K und der Anzahl der Fälle für andere relevante Krankheiten und dividiert durch einen einstellbaren Parameter Rmax.

10. Verfahren nach Anspruch 9, wobei der Vergleich medizinischer Bilder (3) verschiedener Patienten (2) mit vorab geladenen medizinischen Bildern (6) einer Datenbank (4) auf Basis von Ähnlichkeit die folgenden Schritte umfasst:
- die in der Datenbank (4) gespeicherten medizinischen Bilder (6) werden in eine semantische Repräsentation (9) jedes medizinischen Bildes (6) transformiert;
- die zu analysierenden medizinischen Bilder (3) verschiedener Patienten (2) werden in das Tool (1) geladen;
- die zu analysierenden medizinischen Bilder (3) verschiedener Patienten (2) werden in eine semantische Repräsentation (9) umgewandelt; und
- die semantischen Repräsentationen (9) der in der Datenbank (4) gespeicherten medizinischen Bilder (6) und die semantischen Repräsentationen (9) der zu analysierenden medizinischen Bilder (3) verschiedener Patienten (2) werden mittels Kosinusähnlichkeit (10) verglichen.

11. Verfahren nach Anspruch 10, wobei der Schritt der Umwandlung medizinischer Bilder (3, 6) in semantische Repräsentationen (9) die folgenden Schritte umfasst:
- die medizinischen Bilder (3, 6) werden in eine Datenbank (4) oder das Tool (1) geladen;
- die medizinischen Bilder (3, 6) werden mit einem verkürzten tiefen neuronalen Netzwerk DenseNet 121 verarbeitet; und
- die medizinischen Bilder (3, 6) werden durch Average Pooling reduziert, wodurch ein Vektor mit 1024 Komponenten entsteht.

## Revendications

1. Outil (1) d'analyse d'images médicales (3) de patients (2), comprenant une base de données (4) contenant des images médicales (6) préchargées de patients, chaque image préchargée ayant fait l'objet d'un diagnostic, et une interface de saisie permettant de charger plusieurs images médicales (3) de différents patients (2) à analyser,
où cet outil (1) est doté d'un algorithme (16) permettant de prioriser les patients (2) à analyser, dont les images médicales (3) ont été chargées, en fonction de la similarité entre ces images (3) et les images médicales (6) préchargées dans la base de données (4), afin de générer un indice de similarité des maladies (21),
où cet indice de similarité des maladies (21) est utilisé pour classer et prioriser la prise en charge des patients (2),
l'outil (1) se **caractérise par le fait que** le résultat de l'algorithme (16) est un indice de similarité des maladies (21) pour la maladie la plus pertinente,
où ainsi, pour obtenir la maladie la plus pertinente pour une image médicale (3) donnée, Après analyse, l'outil (1) classe les images médicales préchargées (6) et sélectionne les dix cas les plus similaires (17) dans la base de données (4) et pour chacun de ces cas similaires (17), l'outil (1) les classe (18) selon les maladies associées et compte (19) le nombre de cas liés à chaque maladie parmi les dix premiers, la maladie la plus pertinente étant celle qui présente le plus grand nombre de cas,
et l'outil (1) établit un classement par ordre de priorité en fonction du nombre de cas pour la maladie la plus pertinente, pondéré par leur similarité calculée pour chaque image (3) à analyser, multiplié par un coefficient ajustable K et par le nombre de cas pour les autres maladies pertinentes, puis divisé par un paramètre ajustable Rmax.

2. Outil (1) selon la revendication 1, dans lequel le coefficient ajustable K est déterminé par un processus itératif interactif, un sous-ensemble de données préchargées 6 dans la base de données 4 étant choisi comme ensemble de validation 22A et les données préchargées restantes 6 dans la base de données 4 étant utilisées comme ensemble d'entraînement 22B. Initialement, une valeur 22C est choisie pour le coefficient ajustable K, puis l'algorithme 16 est exécuté pour prioriser 220 les images médicales 3 de l'ensemble de validation 22A à l'aide des données de l'ensemble d'entraînement 22B. L'efficacité de la priorisation est ensuite évaluée 22E. Si cette efficacité 22E n'est pas satisfaisante, l'algorithme de priorisation 220 est répété avec une valeur différente 22C pour le coefficient K jusqu'à l'obtention d'une efficacité 22E acceptable.

3. Outil selon la revendication 2, dans lequel le paramètre ajustable Rmax 23A est déterminé, en fonction de la valeur déterminée 22C du coefficient ajustable K, de manière à ce que l'indice de similarité (21) soit limité à une valeur de cent pour cent.

4. Outil (1) selon la revendication 1, dans lequel les images médicales préchargées (6) fournies dans la base de données (4) sont présentes dans celle-ci sous la forme d'une représentation sémantique (9), et les images médicales (3) de différents patients (2) à analyser sont d'abord transformées en une représentation sémantique (9) afin d'être comparées aux représentations sémantiques (9) des images médicales préchargées (6) dans la base de données (4) au moyen de la similarité cosinus (10).

5. Outil (1) selon la revendication 4, dans lequel les représentations sémantiques (9) des images médicales (3) sont obtenues par un réseau neuronal profond DenseNet 121 tronqué et réduites par regroupement moyen, générant un vecteur à 1024 composantes.

6. Outil (1) selon l'une quelconque des revendications précédentes, dans lequel l'outil (1) peut être utilisé pour prioriser les patients en fonction de la gravité plausible d'une maladie donnée.

7. Outil (1) selon la revendication 1, dans lequel les images médicales (3) utilisées par l'outil (1) sont prétraitées (11) par suppression des annotations (12), redimensionnement (13) de l'image à 1000 × 1000 pixels et normalisation (14) par la moyenne et l'écart type.

8. Outil (1) selon la revendication 1, dans lequel l'interface d'entrée est adaptée au chargement d'images médicales (3) à partir d'une plateforme externe distante.

9. Méthode d'analyse d'images médicales (3) de patients (2) utilisables dans un outil (1) selon l'une quelconque des revendications précédentes, ce procédé utilisant une base de données (4) contenant des images médicales (6) préchargées et un algorithme (16) permettant de prioriser les patients (2) à analyser ; ce procédé est utilisé pour prioriser lesdites images médicales (3) de différents patients (2) à analyser et comprend les étapes suivantes :
- chargement des images médicales (3) des patients (2) à prioriser dans l'outil (1) ;
- comparaison de chaque image (3) chargée de différents patients (2) avec les images médicales (6) préchargées afin de déterminer un indice de similarité de la maladie (21). et,
- classement par ordre de priorité de chaque image chargée (3) des patients (2) selon leur indice de similarité de maladie (21),
la méthode étant **caractérisée en ce que** le résultat de l'algorithme (16) est un indice de similarité de maladie (21) pour la maladie la plus pertinente,
où afin d'obtenir la maladie la plus pertinente pour une image médicale (3) à analyser, l'outil (1) classe les images médicales préchargées (6) et sélectionne les dix cas les plus similaires (17) dans la base de données (4). Pour chacun de ces cas similaires (17), l'outil (1) les classe (18) selon les maladies qui leur sont associées et compte (19) le nombre de cas associés à chaque maladie parmi les dix premiers, la maladie la plus pertinente étant celle qui présente le nombre le plus élevé
et l'outil (1) est basé sur le nombre de cas pour la maladie la plus pertinente pondéré par leur similarité calculée pour chaque image (3) à analyser multiplié par un coefficient réglable K et par le nombre de cas pour d'autres maladies pertinentes et divisé par un paramètre réglable Rmax,

10. Méthode selon la revendication 9, dans lequel l'étape de comparaison, par similarité, d'images médicales (3) de différents patients (2) avec des images médicales préchargées (6) d'une base de données (4) comprend les étapes suivantes :
- transformation des images médicales préchargées (6) dans la base de données (4) en une représentation sémantique (9) de chaque image médicale (6) ;
- chargement des images médicales (3) des différents patients (2) à analyser dans l'outil (1) ;
- conversion des images médicales (3) des différents patients (2) à analyser en une représentation sémantique (9) ; et
- comparaison des représentations sémantiques (9) des images médicales préchargées (6) dans la base de données (4) et des représentations sémantiques (9) des images médicales (3) des différents patients (2) à analyser au moyen de la similarité cosinus (10).

11. Méthode selon la revendication 10, dans lequel l'étape de conversion d'images médicales (3, 6) en représentations sémantiques (9) comprend les étapes suivantes :
- les images médicales (3, 6) sont chargées dans une base de données (4) ou l'outil (1) ;
- les images médicales (3, 6) sont traitées par un réseau neuronal profond tronqué DenseNet 121 ; et
- les images médicales (3, 6) sont réduites par regroupement moyen, ce qui génère un vecteur à 1024 composantes.
